# EUROPEAN PATENT SPECIFICATION

(11) **EP 2 550 987 B1**
(45) Date of publication and mention of the grant of the patent: **02.09.2015**
(21) Application number: 12178141.3
(22) Date of filing: 26.07.2012
(51) Int. Cl.: A61M 16/00

(54) **Dialysis machine with haemolysis control**
Dialysemaschine mit Hämolyseüberwachung
Machine de dialyse avec surveillance de l'hémolyse

(30) Priority: 26.07.2011 IT BO20110444
(43) Date of publication of application: 30.01.2013
(73) Proprietor: Bellco S.r.l., Mirandola (IT)
(72) Inventor: Cianciavicchia, Domenico, 64040 Cavuccio (IT)
(74) Representative: Bergadano, Mirko

(56) References cited:
- EP-A1- 1 342 479
- EP-A1- 1 872 812
- US-A- 3 848 580
- US-A- 4 479 760
- US-A1- 2001 020 122
- US-A1- 2010 113 891

## Description

The present invention concerns a dialysis machine with haemolysis control.

Dialysis is a method for cleansing the blood, able to restore the hydrosaline balance and eliminate the excess water and toxic substances that accumulate in the organism during kidney failure, releasing them to a liquid with electrolytic content analogous to that of the normal plasma that does not contain them; here and below said liquid will be indicated by the term "dialysing solution". In application of said method, once the blood has been taken from the patient's arm, it is conveyed through the so-called arterial line and enters the dialyser; at the outlet of the dialyser, the blood is conveyed through the venous line and returns, cleansed, to the patient.

The technique of haemodiafiltration, to which the description refers without loss of generality, entails cleansing of the blood by exploiting the phenomenon of both diffusion and convection. Cleansing by diffusion is due to balancing of the concentrations of the blood and dialysing solution which flow on opposite sides of a semi-permeable membrane, whereas cleansing by convection occurs when a pressure gradient is created between the dialysing solution compartment and the blood compartment, said gradient being favourable to the latter. In this way, via the semi-permeable membrane, there is a passage of plasmatic water and, with it, the toxic substances dissolved therein.

The dialysing solution does not contain the substances to be eliminated from the blood such as urea, uric acid, creatinine, phosphorous etc., but it contains a precise quantity of other substances to be re-balanced such as sodium, calcium, magnesium, potassium etc.

As is known, many of the patients requiring dialysis treatment suffer from massive haemolysis, a pathology involving destruction of the red blood cells greater than the physiological destruction of 1-2% per day of the population of red blood cells.

For these patients it is therefore particularly important for haemolysis in the blood to be monitored also during dialysis.

The European Patent Application EP1342479 A(1) describes an assembly for controlling and regulating flow of a dialysis solution in a haemodiafiltration process, having a first and a second pump located respectively along a dialysis solution inlet branch and outlet branch of a haemodialysis filter; a differential flowmeter for reading the difference in flow of the dialysis solution in and out of the filter; and a central control unit for receiving information from the differential flowmeter, and making a calculated variation in the delivery of the pump, so as to periodically regulate the pressure of the dialysis solution in the filter, and impose alternate calculated flow of the dialysis solution and the plasma water through the membrane of the filter.

The US Patent Application US2001/020122 A(1) describes systems and methods for noninvasively measuring the levels of urea, blood osmolarity (or Na+), plasma free hemoglobin and tissue water content in a patient's blood or tissue. Light of selected wavelengths is passed through blood or body tissue and the transmitted or reflected light is detected and the detected signals can be electronically compared and manipulated to provide the non-invasive, continuous and quantitative display of a patient's blood urea, blood osmolarity (or Na+), plasma free hemoglobin and tissue water content.

The object of the present invention is to produce a dialysis machine, the technical characteristics of which are such as to ensure control of the haemolysis value in the patient's blood during dialysis treatment.

The subject of the present invention is a dialysis machine as claimed in Claim 1.

According to a first preferred embodiment of the dialysis machine of the present invention, the measuring device is applied on said arterial line.

According to a second preferred embodiment of the dialysis machine of the present invention, the measuring device is applied on said venous line.

According to a third preferred embodiment, the dialysis machine of the present invention comprises an isolated ultrafiltration device arranged along the arterial line of the patient's blood, from which a branch starts suitable for conveying the components of the blood and on which the haemoglobin concentration measuring device is applied.

The following examples are provided for illustrative nonlimiting purposes, for a better understanding of the invention with the help of the figures of the attached drawings, in which:
- figure 1 is a schematic view of part of a first preferred embodiment of the dialysis machine according to the present invention; and
- figure 2 is a schematic view of part of a second preferred embodiment of the dialysis machine according to the present invention.

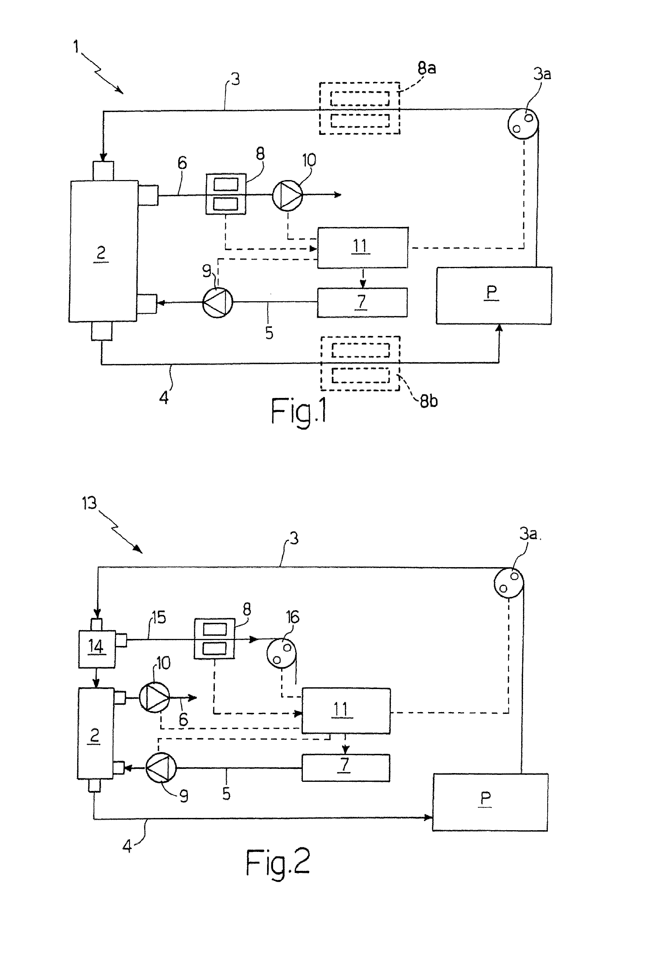

In figure 1, the number 1 indicates overall a first preferred embodiment of the dialysis machine (illustrated only partially) according to the present invention.

The machine 1 comprises a dialysis filter 2 (known and not described in detail), an arterial line 3 which is responsible for conveying the blood from a patient P to the filter 2, a pump 3a applied to the arterial line 3 to guarantee movement of the blood, a venous line 4 responsible for conveying the blood from the filter 2 to the patient P, an inlet branch 5 and an outlet branch 6 of a dialysing solution into and out of the filter 2, a preparation device 7 for the dialysing solution connected to the inlet branch 5, a device for measuring the concentration of haemoglobin 8 applied to the outlet branch 6, a pair of pumps 9 and 10 applied to the inlet branch 5 and to the outlet branch 6 of the dialysing solution respectively, and a command and control unit 11.

The command and control unit 11 is connected to the haemoglobin concentration measuring device 8 to receive the data from it.

Figure 1 shows two possible variations in which the haemoglobin concentration measuring device is indicated by a broken line. In particular, in a first possible variation, a measuring device indicated by 8a is applied along the arterial line 3, while in a second possible variation, a measuring device indicated by 8b is applied along the venous line 4.

In figure 2, the number 13 indicates as a whole a second preferred embodiment of the dialysis machine (illustrated only partially) according to the present invention. The parts of the machine 13 equal to those of the machine 1 will be indicated by the same numbering and will not be described again.

The machine 13 differs from the machine 1 substantially to comprise an isolated ultrafiltration device 14 arranged along the arterial line 3. The isolated ultrafiltration device 14 can produce plasmatic water or plasma depending on whether it is a haemofilter or a plasma filter respectively. The plasmatic water or the plasma produced flow into a branch line 15, to which a pump 16 is applied. Generally, the branch line 15 comprises an adsorption cleansing device and re-connects to the arterial line 3 in the section between the isolated ultrafiltration device 14 and the haemodialysis filter 2.

As illustrated in figure 2, the machine 13 entails application of the haemoglobin concentration measuring device 8 on the branch line 15 and connection, as for the machine 1, to the command and control unit 12.

The measuring device 8 is an optical sensor and, as may be obvious from a study of the present invention, the type of measuring device is in no way binding and therefore cannot be limiting.

The dialysis machine subject of the present invention allows continuous monitoring of the haemoglobin concentration in the blood of the patient undergoing dialysis.

If the haemolysis sensor reveals an abnormal concentration of haemoglobin, the machine not only signals the abnormality but also acts on the pumping systems to lower the pressures in the various points of the extracorporeal circuit. In particular, the control unit 11, once it has recorded an abnormal haemolysis value, activates an intervention system (not illustrated for the sake of simplicity) both to signal the abnormal haemoglobin value and to modify the pressure regimes acting on the pumping systems.

## Claims

1. A dialysis machine (1; 13) comprising a haemodialysis filter (2), an inlet branch (5) for letting a dialysing solution into said filter (2), an outlet branch (6) for letting the dialysing solution out of said filter (2), an arterial line (3), which is responsible for the transport of blood from a patient (P) to the filter (2), a venous line (4), which is responsible for the transport of blood from the filter (2) to the patient (P), and a plurality of pumps (3a, 9, 10, 16), which are suited for circulating both the blood and the dialysing solution, and an haemolysis measuring device (8; 8a; 8b), said haemolysis measuring device (8; 8a; 8b) being arranged to operate on a line (4; 3; 15) through which the blood or a component thereof flows or on said outlet branch (6) of the dialysing solution; said haemolysis measuring device (8; 8a; 8b) being a device for the measurement of the concentration of haemoglobin and being an optical sensor; the dialysis machine further comprises a command and control unit (11) connected to said haemolysis measuring device (8; 8a; 8b) to receive the haemolysis data from it; said dialysis machine being **characterised in that** the control unit (11) is further connected to an intervention system and **in that** the control unit (11) activates the intervention system once it has recorded an abnormal haemolysis value to signal the abnormal haemoglobin value and to modify the pressure regimes acting on the pumps (3a, 9, 10, 16).

2. Machine as claimed in claim 1, **characterised in that** said measuring device (8a) is applied to said arterial line 3.

3. Machine as claimed in claim 1, **characterised in that** said measuring device (8b) is applied to said venous line (4).

4. Machine as claimed in claim 1, **characterised in that** it comprises an isolated ultrafiltration device (14) arranged along the arterial line (3) of the patient's blood, and from which a branch line (15) starts to which said measuring device (8) is applied, said branch line (15) containing the circulation of plasma or plasmatic water.

## Patentansprüche

1. Dialysemaschine (1; 13), die Folgendes aufweist: einen Hämodialyse-Filter (2), einen Einlasszweig (5) zum Einlassen einer Dialyselösung in den Filter (2), einen Auslasszweig (6) zum Auslassen der Dialyselösung aus dem Filter (2), eine arterielle Leitung (3), die für den Transport von Blut von einem Patienten (P) zu dem Filter (2) zuständig ist, eine venöse Leitung (4), die für den Transport von Blut vom Filter (2) zu dem Patienten (P) zuständig ist, und eine Vielzahl von Pumpen (3a, 9, 10, 16), die zum Zirkulieren von sowohl dem Blut als auch der Dialyselösung geeignet sind, und eine Hämolyse-Messvorrichtung (8; 8a; 8b), wobei die Hämolyse-Messvorrichtung (8; 8a; 8b) so angeordnet ist, dass sie an einer Leitung (4; 3; 15) arbeitet, durch welche das Blut oder eine Komponente davon fließt oder an dem Auslasszweig (6) der Dialyselösung; wobei die Hämolyse-Messvorrichtung (8; 8a; 8b) eine Vorrichtung für die Messung der Hämoglobin-Konzentration ist und wobei sie ein optischer Sensor ist; wobei die Dialysemaschine ferner eine Befehls- und Steuereinheit (11) aufweist, die mit der Hämolyse-Messvorrichtung (8; 8a; 8b) verbunden ist, um die Hämolyse Daten von ihr zu erhalten, wobei die Dialysemaschine **dadurch gekennzeichnet ist, dass** die Steuereinheit (11) ferner mit einem Interventionssystem verbunden ist und dass die Steuereinheit (11) das Interventionssystem aktiviert, sobald sie einen abnormen Hämolysewert aufzeichnet, um den abnormen Hämoglobinwert anzuzeigen und um die Druckgrößen, die auf bzw. von den Pumpen (3a; 9, 10, 16) wirken, zu modifizieren.

2. Maschine nach Anspruch 1, **dadurch gekennzeichnet, dass** die Messvorrichtung (8a) auf die arterielle Leitung (3) angewandt wird.

3. Maschine nach Anspruch 1, **dadurch gekennzeichnet, dass** die Messvorrichtung (8b) auf die venöse Leitung (4) angewandt wird.

4. Maschine nach Anspruch 1, **dadurch gekennzeichnet, dass** sie eine isolierte Ultrafiltrationsvorrichtung (14) aufweist, die entlang der arteriellen Leitung (3) des Blutes des Patienten angeordnet ist, und von der aus eine Zweigleitung (15) beginnt, an der die Messvorrichtung (8) angewandt wird, wobei die Zweigleitung (15) die Zirkulation von Plasma oder plasmatischem Wasser umfasst.

## Revendications

1. Machine de dialyse (1 ; 13) comprenant un filtre d'hémodialyse (2), un embranchement d'entrée (5) pour laisser entrer une solution de dialyse dans ledit filtre (2), un embranchement de sortie (6) pour laisser sortir la solution de dialyse dudit filtre (2), une voie artérielle (3), qui est chargée du transport de sang d'un patient (P) jusqu'au filtre (2), une voie veineuse (4), qui est chargée du transport de sang du filtre (2) jusqu'au patient (P), et une pluralité de pompes (3a, 9, 10, 16), qui sont adaptées à la circulation du sang et de la solution de dialyse, et un dispositif de mesure d'hémolyse (8 ; 8a ; 8b), ledit dispositif de mesure d'hémolyse (8 ; 8a ; 8b) étant agencé pour agir sur une voie (4 ; 3 ; 15) par laquelle le sang ou un composant du sang s'écoule ou agir sur ledit embranchement de sortie (6) de la solution de dialyse ; ledit dispositif de mesure d'hémolyse (8 ; 8a ; 8b) étant un dispositif servant à mesurer la concentration d'hémoglobine et étant un capteur optique ; la machine de dialyse comprenant, en outre, une unité de commande et de contrôle (11) reliée audit dispositif de mesure d'hémolyse (8 ; 8a ; 8b) pour recevoir du dispositif les données d'hémolyse ; ladite machine de dialyse étant **caractérisée en ce que** l'unité de commande (11) est, en outre, reliée à un système d'intervention et **en ce que** l'unité de commande (11) actionne le système d'intervention une fois qu'il a enregistré une valeur d'hémolyse anormale afin de signaler la valeur d'hémolyse anormale et de modifier les régimes de pression agissant sur les pompes (3a, 9, 10, 16).

2. Machine selon la revendication 1, **caractérisée en ce que** ledit dispositif de mesure (8a) est appliqué à ladite voie artérielle (3).

3. Machine selon la revendication 1, **caractérisée en ce que** ledit dispositif de mesure (8b) est appliqué à ladite voie veineuse (4).

4. Machine selon la revendication 1, **caractérisée en ce qu'**elle comprend un dispositif d'ultrafiltration isolé (14) agencé le long de la voie artérielle (3) du sang du patient, et à partir duquel part une voie d'embranchement (15) à laquelle le dispositif de mesure (8) est appliqué, ladite voie d'embranchement (15) contenant la circulation de plasma ou d'eau plasmatique.
